# EUROPEAN PATENT SPECIFICATION

(11) **EP 2 479 213 B1**
(45) Date of publication and mention of the grant of the patent: **13.02.2019**
(21) Application number: 10817145.5
(22) Date of filing: 14.09.2010
(51) Int. Cl.: C08L 33/04, A61K 8/19, A61K 8/27, A61K 8/29, A61K 8/81, C08F 2/44, C08J 3/12, C08K 3/22

(54) **RESIN POWDER THAT CONTAINS METAL OXIDE ENCAPSULATED THEREIN, LIQUID DISPERSION AND AQUEOUS DISPERSION THAT CONTAIN SAME, PROCESS FOR PRODUCTION OF RESIN POWDER THAT CONTAINS METAL OXIDE ENCAPSULATED THEREIN, AND COSMETICS**
HARZPULVER MIT DARIN EINGEKAPSELTEM METALLOXID, FLÜSSIGE DISPERSION UND WÄSSRIGE DISPERSION DAMIT, VERFAHREN ZUR HERSTELLUNG DES HARZPULVERS MIT DARIN EINGEKAPSELTEM METALLOXID SOWIE KOSMETIKARTIKEL DAMIT
POUDRE DE RÉSINE QUI CONTIENT DE L'OXYDE MÉTALLIQUE ENCAPSULÉ DANS CELLE-CI, DISPERSION LIQUIDE ET DISPERSION AQUEUSE LA CONTENANT, PROCÉDÉ POUR LA PRODUCTION DE POUDRE DE RÉSINE QUI CONTIENT DE L'OXYDE MÉTALLIQUE ENCAPSULÉ DANS CELLE-CI ET PRODUITS COSMÉTIQUES

(30) Priority: 15.09.2009 JP 2009213235
(43) Date of publication of application: 25.07.2012
(73) Proprietor: Sumitomo Osaka Cement Co., Ltd., Tokyo 102-8465 (JP)
(72) Inventor: MATSUSHITA Hirokazu, Tokyo 102-8465 (JP); HOSODA Shingo, Tokyo 102-8465 (JP); KATAYAMA Chiaki, Tokyo 102-8465 (JP)
(74) Representative: Cabinet Laurent & Charras
(86) International application number: PCT/JP2010/065775
(87) International publication number: WO 2011/034032

(56) References cited:
- EP-A1- 1 291 370
- EP-A1- 1 388 550
- EP-A1- 1 760 119
- WO-A1-2008/032859
- JP-A- 9 208 437
- JP-A- 9 208 864
- JP-A- 2003 073 407
- JP-A- 2007 008 833
- JP-A- 2008 239 897
- JP-A- 2009 155 622

## Description

### Technical Field

The present invention relates to a method of manufacturing metallic oxide particle-containing resin powder. The metallic oxide particle-containing resin powder is preferably used for a variety of cosmetic products, such as skin care cosmetic products, makeup cosmetic products, and body care cosmetic products, particularly for whitening by skin care cosmetic products for which a UV shielding capability is required, base makeup by makeup cosmetic products, and sunscreening by body care cosmetic products, a dispersion liquid and an aqueous dispersion element including the same.

Priority is claimed on Japanese Patent Application No. 2009-213235, filed on September 15, 2009, the content of which is incorporated herein by reference.

### Background Art

In the past, metallic oxides were added to cosmetic products as a pigment for providing a specific color hue, functional materials for providing UV shielding properties, IR shielding properties, and antibacterial functions, and the like. For instance, document EP 1 388 550 discloses composite particles comprising a core made of metal oxide, coated with a layer a methacrylate polymer. Document EP 1 291 370 discloses composite particles of the core-shell type, the core containing a titanium dioxide particles or zinc oxide particles, having a stearic acid surface treatment.

Among them, examples of the metallic oxides, which are inorganic UV shielding materials that shield a wide wavelength range of ultraviolet rays including near ultraviolet rays, include zinc oxide, titanium oxide, which are important white pigments as well. For instance, document WO 2008/032859 discloses composite particles comprising a core made of a UV shielding material and a coating made of polymer. The metallic oxides can be made to allow a majority of visible light rays to pass through, without absorbing them, and absorb a majority of ultraviolet rays by setting the dispersion particle diameter to 0.1 µm or less when the metallic oxide is dispersed in a base material.

Document EP 1 760 119 discloses a method for producing metallic oxide complex powder in which a polymerizable and unsaturated group-containing monomer is polymerized via a polymerization initiation group introduced onto the surface of the metallic oxide.

In addition, since zinc oxide, which is particularly added to sunscreens among cosmetic products so as to exhibit effects, is an amphoteric oxide, zinc oxide is dissolved in water only to a small amount.

Since zinc oxide has been known to absorb an oil component, and zinc ions generated when zinc oxide is dissolved in water react with a fatty acid so as to generate metallic soap, zinc oxide is used as an absorbent for sebum produced from the skin and a deodorant that absorbs a body odor component. Furthermore, since zinc ions have physiological actions, zinc oxide also has been used as an astringent agent.

As powder used for cosmetic materials having UV shielding properties, spherical resin powder which includes at least one of zinc oxide, titanium oxide, and cerium oxide in a resin at 1% by mass to 80% by mass of the total weight, has a particle diameter of 30 µm or less, and has a UV shielding function (Patent Document 1), resin powder obtained by dispersing a metallic oxide having a UV shielding function in a resin monomer and suspension-polymerizing or emulsion-polymerizing the mixture (Patent Document 2), and the like are proposed.

However, since zinc oxide or titanium oxide of the related art, which shielded a wide wavelength range of ultraviolet rays including near ultraviolet rays, had a large refractive index of 1.9 for zinc oxide and of 2.5 for titanium oxide, when the dispersion particle diameter became 0.1 µm or more in a case in which zinc oxide or titanium oxide was included in cosmetic material, there were problems in that the cosmetic material became whitened so as to impair the transparency, and natural makeup was not possible.

In order to solve the problem, it is necessary to set the dispersion particle diameter of zinc oxide or titanium oxide to 0.1 µm or less; however, in order to set the dispersion particle diameter of zinc oxide or titanium oxide to 0.1 µm or less, an advanced dispersion technique is required, which is extremely difficult to be achieved in current circumstances. Furthermore, there is a concern that the fine particles of the zinc oxide or titanium oxide may be absorbed into the body through sweat glands on the skin, which may cause another concern in terms of the safety to human bodies.

On the other hand, in a case in which the particle diameter of zinc oxide or titanium oxide was set to 1 µm or more, when zinc oxide or titanium oxide was used for a cosmetic material, since the transparency was impaired, and, simultaneously, roughness and the like were caused such that the feeling was deteriorated, there was a problem in that it was difficult to use zinc oxide or titanium oxide for a cosmetic material.

In addition, zinc oxide is soluble in water at a small amount, and is sometimes used as a deodorant or astringent agent that absorbs a body odor component through an action by zinc ions eluted from the zinc oxide; however, since there is a concern that the simultaneously eluted zinc ions may react with other components of the cosmetic product, such as an oil solution, a perfume, a colorant, an organic UV absorbing agent, and a water-soluble polymer, so as to induce a change in the viscosity, occurrence of abnormal odor, discoloration, coloration, gelatification, and the like, there is another problem in that it is not possible to increase the content of water in the cosmetic material, and the degree of freedom in formulating a cosmetic material is degraded.

### Citation List

### Patent Document

[Patent Document 1] Japanese Patent No. 3469641
[Patent Document 2] Japanese Patent No. 3205249

### Summary of Invention

### Technical Problem

The problem to be solved is that the dispersion element of cosmetic materials and the like of the related art, which includes metallic oxide particles of zinc oxide, titanium oxide, or the like that shields ultraviolet rays, is whitened so as to impair the transparency when the dispersion particle diameter is 0.1 µm or more such that the dispersion element cannot allow a majority of visible light rays to pass through, without absorbing them, and, particularly, the degree of freedom in formulating a cosmetic material is degraded.

### Solution to Problem

As a result of repeating thorough studies for solving the problem, the present inventors found that, when a (meth)acrylic-based resin is made to contain metallic oxide particles having a UV shielding function so as to produce resin powder, the average particle diameter of the resin powder is set to 0.1 µm to 1 µm, the metallic oxide particles are made to include one or two or more selected from a group of zinc oxide, titanium oxide, cerium oxide, and iron oxide, and to have an average particle diameter of 0.003 µm to 0.1 µm, the content of the metallic oxide particles in the resin powder is set to 1% by mass to 80% by mass, and the metallic oxide particles are dispersed in the resin powder without being exposed on the surface of the resin powder, it is possible to produce resin powder that is excellent in the absorbing properties of ultraviolet rays, and allows a majority of visible light rays to pass through, without absorbing them, so as to be excellent in terms of the transparency. In addition, even in a case in which the resin powder is used for a cosmetic material, the cosmetic material is excellent in terms of the transparency, and roughness and the like are not caused so that the sensation during use is excellent, and completed the invention.

That is, the method of manufacturing metallic oxide particle-containing resin powder according to the method of the invention yields resin powder in which metallic oxide particles having a UV shielding function are contained in a (meth)acrylic-based resin, wherein the average particle diameter of the resin powder is 0.1 µm to 1 µm, the metallic oxide particles are particles that include one or two or more selected from a group of zinc oxide, titanium oxide, cerium oxide, and iron oxide, and have an average particle diameter of 0.003 µm to 0.1 µm, the content of the metallic oxide particles in the resin powder is 1% by mass to 80% by mass, and the metallic oxide particles are dispersed in the resin powder without being exposed on the surface of the resin powder.

The amount of metal elements eluted into pure water in a case in which the resin powder is immersed in pure water is preferably 0.05 ppm or less, and the amount of metal elements eluted into an acetic acid aqueous solution in a case in which the resin powder is immersed in an acetic acid aqueous solution is preferably 1.5 ppm or less.

The metallic oxide particle-containing resin powder dispersion liquid is a dispersion liquid obtained by dispersing the metallic oxide particle-containing resin powder obtained with the method of the invention in a dispersion medium, in which, when the content of the metallic oxide particle-containing resin powder in the dispersion liquid is adjusted to be 5% by mass, the transmittance T₆₀₀ of the adjusted dispersion liquid with respect to light rays having a wavelength of 600 nm is preferably 60% or more, the ratio T₃₇₅/T₆₀₀ of the transmittance T₃₇₅ with respect to light rays having a wavelength of 375 nm to the transmittance T₆₀₀ with respect to light rays having a wavelength of 600 nm is preferably 0.3 or less, and the ratio T₃₅₀/T₆₀₀ of the transmittance T₃₅₀ with respect to light rays having a wavelength of 350 nm to the transmittance T₆₀₀ with respect to light rays having a wavelength of 600 nm is preferably 0.15 or less.

The metallic oxide particle-containing resin powder aqueous dispersion element contains 1% by mass to 80% by mass of the metallic oxide particle-containing resin powder obtained with the method of the invention and 5% by mass to 20% by mass of an alcohol.

Furthermore, the metallic oxide particle-containing resin powder aqueous dispersion element may contain 0.001% by mass to 10% by mass of a water-soluble polymer.

In a method of manufacturing metallic oxide particle-containing resin powder of the invention, metallic oxide particles having an average particle diameter of 0.003 µm to 0.1 µm and a UV shielding function are dispersed in a (meth)acrylic-based resin monomer including 1% by mass to 50% by mass of a dispersant with respect to the metallic oxide particles so as to produce a (meth)acrylic-based resin monomer dispersion liquid, subsequently, the (meth)acrylic-based resin monomer dispersion liquid is suspended or emulsified in pure water including 0.1% by mass to 10% by mass of a suspension protecting agent, 0.01% by mass to 5% by mass of a silicone-based antifoam agent, and 0.1% by mass to 10% by mass of a cross-linking agent with respect to the (meth)acrylic-based resin monomer dispersion liquid so as to produce a suspension liquid or emulsified liquid, and, subsequently, 0.01% by mass to 1% by mass of a polymerization initiator with respect to the suspension liquid or emulsified liquid is added to the suspension liquid or emulsified liquid so as to cause suspension polymerization or emulsification polymerization, thereby generating metallic oxide particle-containing resin powder.

A cosmetic material is obtained by containing 1% by mass to 50% by mass, in terms of the metallic oxide particle-containing resin powder, of one or two or more selected from a group of the metallic oxide particle-containing resin powder obtained with the method of the invention, the metallic oxide particle-containing resin powder dispersion liquid, and the metallic oxide particle-containing resin powder aqueous dispersion element.

### Advantageous Effects of Invention

According to the method of manufacturing metallic oxide particle-containing resin powder according to the method of the invention, since the average particle diameter of the resin powder is set to 0.1 µm to 1 µm, the metallic oxide particles are made to include one or two or more selected from a group of zinc oxide, titanium oxide, cerium oxide, and iron oxide, and to have an average particle diameter of 0.003 µm to 0.1 µm, the content of the metallic oxide particles in the resin powder is set to 1% by mass to 80% by mass, and the metallic oxide particles are dispersed in the resin powder without being exposed on the surface of the resin powder, it is possible to make the powder into fine particles, improve the UV absorbing performance, and improve the transparency with respect to visible light rays.

In addition, since the metallic oxide particles are not exposed on the surface of the resin powder, it is possible to suppress elution of a metallic oxide, which is a component of the particles, into a solvent even in a case in which the resin powder is dispersed in the solvent. Therefore, in a case in which the resin powder is used to formulate cosmetic products, the resin powder can be formulated not only for water-in-oil type (W/O type) cosmetic products but also for oil-in-water type (O/W type) cosmetic products, and it is possible to further increase the degree of freedom in formulating a cosmetic product.

In addition, since the average particle diameter of the resin powder is set to 0.1 µm to 1 µm, even in a case in which the resin powder is used for a cosmetic product, roughness and the like are not caused, and the sensation during use is excellent.

According to the metallic oxide particle-containing resin powder dispersion liquid, since the transmittance T₆₀₀ of the adjusted dispersion liquid with respect to light rays having a wavelength of 600 nm is set to 60% or more, the ratio T₃₇₅/T₆₀₀ of the transmittance T₃₇₅ with respect to light rays having a wavelength of 375 nm to the transmittance T₆₀₀ with respect to light rays having a wavelength of 600 nm is set to 0.3 or less, and the ratio T₃₅₀/T₆₀₀ of the transmittance T₃₅₀ with respect to light rays having a wavelength of 350 nm to the transmittance T₆₀₀ with respect to light rays having a wavelength of 600 nm is set to 0.15 or less, it is possible to improve the transparency with respect to visible light rays.

According to the metallic oxide particle-containing resin powder aqueous dispersion element, since the metallic oxide particle-containing resin powder aqueous dispersion element contains 1% by mass to 80% by mass of the metallic oxide particle-containing resin powder obtained with the method of the invention and 5% by mass to 20% by mass of an alcohol, it is possible to improve the transparency with respect to visible light rays.

According to the method of manufacturing metallic oxide particle-containing resin powder of the invention, it is possible to easily manufacture the metallic oxide particle-containing resin powder.

According to the cosmetic material, since the cosmetic material contains 1% by mass to 50% by mass, in terms of the metallic oxide particle-containing resin powder, of one or two or more selected from a group of the metallic oxide particle-containing resin powder obtained with the method of the invention, the metallic oxide particle-containing resin powder dispersion liquid, and the metallic oxide particle-containing resin powder aqueous dispersion element, there is no concern of whitening, and it is possible to sufficiently secure the transparency. In addition, roughness and the like are not caused, and the sensation during use is excellent.

### Brief Description of Drawings

FIG. 1 is a view showing the volume particle size distribution and cumulative volume particle size distribution of a resin monomer dispersion liquid of Example 1.
FIG. 2 is a view showing the volume particle size distribution and cumulative volume particle size distribution of an emulsion A of Example 1.
FIG. 3 is a view showing the volume particle size distribution and cumulative volume particle size distribution of an emulsion B of Example 1.
FIG. 4 is a view showing the volume particle size distribution and cumulative volume particle size distribution of an emulsion C of Example 1.
FIG. 5 is a view showing the volume particle size distribution and cumulative volume particle size distribution of zinc oxide particle-containing resin powder A of Example 1.
FIG. 6 is a view showing the volume particle size distribution and cumulative volume particle size distribution of zinc oxide particle-containing resin powder B of Example 1.
FIG. 7 is a view showing the volume particle size distribution and cumulative volume particle size distribution of zinc oxide particle-containing resin powder C of Example 1.
FIG. 8 is a transmission electron microscope (TEM) image of zinc oxide particle-containing resin powder B of Example 1.
FIG. 9 is a scanning electron microscope (SEM) image of zinc oxide particle-containing resin powder B of Example 1.
FIG. 10 is a view respectively showing the spectral transmittances of dispersion liquids A to C of Example 1 and dispersion liquids D and E of Comparative Example 1 and 2.
FIG. 11 is a view showing the volume particle size distribution and cumulative volume particle size distribution of a resin monomer dispersion liquid of Example 2.
FIG. 12 is a view showing the volume particle size distribution and cumulative volume particle size distribution of an emulsion E of Example 2.
FIG. 13 is a view showing the volume particle size distribution and cumulative volume particle size distribution of titanium oxide particle-containing resin powder E of Example 2.
FIG. 14 is a transmission electron microscope (TEM) image of titanium oxide particle-containing resin powder E of Example 2.
FIG. 15 is a scanning electron microscope (SEM) image of titanium oxide particle-containing resin powder E of Example 2.
FIG. 16 is a view respectively showing the spectral transmittances of zinc oxide particle-containing resin powder aqueous dispersion elements of Examples 6 and 7.
FIG. 17 is a view respectively showing changes in the viscosities of aqueous dispersion elements of Example 6 and Comparative Example 3.
FIG. 18 is a view respectively showing changes in the viscosities of aqueous dispersion elements of Example 7 and Comparative Example 4.

### Description of Embodiments

Embodiments of the metallic oxide particle-containing resin powder, the dispersion liquid and the aqueous dispersion element including the same, the method of manufacturing metallic oxide particle-containing resin powder, and the cosmetic material will be described.

Meanwhile, the embodiments will be described to specifically explain the invention for better understanding of the purport of the invention, and do not limit the invention unless otherwise described.

### [Metallic oxide particle-containing resin powder]

The metallic oxide particle-containing resin powder of the present embodiment is resin powder in which metallic oxide particles having an average particle diameter of 0.003 µm to 0.1 µm and a UV shielding function are contained in a (meth)acrylic-based resin the average particle diameter of the resin particles is 0.1 µm to 1 µm, and the metallic oxide particles are dispersed in the resin powder without being exposed on the surface of the resin powder.

Here, the state of the metallic oxide particles being dispersed in the resin powder without being exposed on the surface of the resin powder refers to a state in which the metallic oxide particles dispersed in the resin powder are completely implanted in the resin powder, and, furthermore, the surface of the resin powder is completely coated with the resin so that the metallic oxide particles are not exposed on the surface of the coated resin.

In the metallic oxide particle-containing resin powder, the amount of zinc (Zn) eluted is small in spite of the small average particle diameter. Furthermore, the amount of zinc (Zn) eluted decreases as the average particle diameter of the resin powder decreases (refer to Tables 1 and 2 as described below) .

Generally, the amount of zinc eluted tends to increase as the particle diameter of the powder decreases, that is, the specific surface area of the resin powder increases. Therefore, in the metallic oxide particle-containing resin powder, since the amount of zinc (Zn) eluted can be suppressed to be small in spite of the large specific surface area, it can be considered to sufficiently coat around the zinc oxide particles, and, furthermore, prevent the zinc oxide fine particles from being exposed on the surface of the metallic oxide particle-containing resin powder.

A resin obtained by polymerizing one or two or more selected from a group of resins of acrylic acid esters, methacrylic acid esters, acrylic styrene copolymers, acrylamide copolymers, acrylic epoxy copolymers, acrylic urethane copolymers, acrylic polyester polymers, and silicon acrylic copolymers is preferably used as the (meth) acrylic-based resin.

Examples of the (meth) acryl-based monomers include methyl acrylate, ethyl acrylate, propyl acrylate, n-butyl acrylate, isobutyl acrylate, hexyl acrylate, 2-ethylhexyl acrylate, n-octyl acrylate, dodecyl acrylate, lauryl acrylate, stearyl acrylate, 2-chloroethyl acrylate, phenyl acrylate, methyl α-chloroacrylate, trifluoroethyl acrylate, tetrafluoropropyl acrylate, methyl methacrylate, ethyl methacrylate, propyl methacrylate, n-butyl methacrylate, isobutyl methacrylate, hexyl methacrylate, 2-ethylhexyl methacrylate, n-octyl methacrylate, dodecyl methacrylate, lauryl methacrylate, and stearyl methacrylate.

In addition, examples of a monomer that is polymerized with the (meth)acryl-based monomer include styrene, o-methylstyrene, m-methylstyrene, p-methylstyrene, α-methylstyrene, o-ethylstyrene, m-ethylstyrene, p-ethylstyrene, 2,4-dimethylstyrene, p-n-butylstyrene, p-t-butylstyrene, p-n-hexylstyrene, p-n-octylstyrene, p-n-nonylstyrene, p-n-decylstyrene, p-dodecylstyrene, p-methoxystyrene, p-phenylstyrene, p-chlorostyrene, 3,4-dichlorostyrene, vinyl acetate, vinyl propionate, vinyl benzoate, vinyl butyrate, N-vinylpyrrolidone, vinylidene fluoride, tetrafluoroethylene, hexafluoropropylene, butadiene, and isoprene.

The monomer may be polymerized singly or polymerized in combination of two or more with the (meth)acryl-based monomer.

The metallic oxide particles are particles that include one or two or more selected from a group of zinc oxide, titanium oxide, cerium oxide, and iron oxide and have a UV shielding function, in which the average particle diameter is 0.003 µm to 0.1 µm, more preferably 0.01 µm to 0.05 µm, and most preferably 0.02 µm to 0.04 µm.

When the average particle diameter of the metallic oxide particles is less than 0.003 µm, the degree of crystallinity is degraded such that the metallic oxide particles no longer develop the UV shielding function. In addition, when the average particle diameter exceeds 0.1 µm, since the scattering coefficient of the particles with respect to visible light rays increases, the transparency is significantly degraded, consequently, the light transmission is significantly degraded, and the transparency is deteriorated, which is not preferred.

Metallic oxide particles whose surfaces are treated with organopolysiloxane may be used as the metallic oxide particles.

The content of the metallic oxide particles in the resin powder is preferably 1% by mass to 80% by mass, more preferably 30% by mass to 80% by mass, and most preferably 50% by mass to 80% by mass.

Here, when the content of the metallic oxide particles in the resin powder is less than 1% by mass, the amount of the metallic oxide particles is too small, and the UV shielding function of the metallic oxide particles cannot be sufficiently developed. Therefore, a large amount of the resin powder is required to sufficiently develop the UV shielding function, and material design becomes extremely difficult when manufacturing cosmetic products. On the other hand, when the content exceeds 80% by mass, the amount of the metallic oxide particles increases relative to the resin, and, as a result, the dispersibility of the metallic oxide particles in the resin is degraded, and the homogeneity of the composition is impaired, which is not preferred.

The diameter of the metallic oxide particles dispersed in the metallic oxide particle-containing resin powder is preferably 0.1 µm or less, more preferably 0.05 µm or less, and most preferably 0.03 µm or less.

Here, when the dispersion particle diameter of the metallic oxide particles in the resin powder exceeds 0.1 µm, the scattering coefficient of the resin powder with respect to visible light rays increases, consequently, the transparency is significantly degraded, and there is a concern of devitrification depending on circumstance, which is not preferred.

The average particle diameter of the resin powder is preferably 0.1 µm to 1 µm, more preferably 0.2 µm to 0.8 µm, and most preferably 0.3 µm to 0.6 µm.

Here, when the average particle diameter of the resin powder is less than 0.1 µm, a dewatering treatment becomes difficult such that the resin powder becomes liable to be agglomerated, as a result, the agglomerating properties of the resin powder become strong such that the dispersibility is degraded, and it becomes impossible to sufficiently develop the UV shielding function. On the other hand, when the average particle diameter exceeds 1 µm, in a case in which the resin powder is used as a cosmetic material, the spreadability or lubricity on the skin is degraded, as a result, roughness and the like are caused such that skin feeling and the like are deteriorated, and the sensation during use becomes poor, which is not preferred.

The amount of metal elements eluted into pure water in a case in which the resin powder is immersed in pure water is 0.05 ppm or less, and preferably 0.01 ppm or less, and the amount of metal elements eluted into an acetic acid aqueous solution in a case in which the resin powder is immersed in an acetic acid aqueous solution is 1.5 ppm or less, and preferably 1.0 ppm or less.

Here, the reason why the amounts of the metal elements eluted into pure water and the acetic acid aqueous solution are limited to the above ranges is that elution of the metallic oxide, which is a component of the resin powder, into a solvent, such as pure water, can be suppressed in this range in a case in which the resin powder is used as a cosmetic material. When the amount of the metal elements eluted is limited to the above range, the eluted metal ions react with other components of the cosmetic material so that troubles, such as a change in the components or discoloration, can be prevented.

For the resin powder, according to necessity, the surface may be treated with 1% by mass to 20% by mass of organosiloxane with respect to the resin powder.

Examples of the organosiloxane include dialkyl alkoxy silane compounds. Among them, organopolysiloxane or a modified organopolysiloxane obtained by modifying an organopolysiloxane with one or two or more selected from a group of an alkyl group, an isocyanate group, an epoxy group, an acrylic group, and an alkyl silicon compound is preferably used, and, particularly, dimethylpolysiloxane (silicone oil) and a modified dimethylpolysiloxane obtained by modifying the dimethylpolysiloxane (silicone oil) (modified silicone oil) are preferably used.

When the surface of the resin powder is treated with organosiloxane, elution of the metallic oxide, which is a component of the resin powder, into a solvent, such as pure water, can be further suppressed.

### [Metallic oxide particle-containing resin powder dispersion liquid]

The metallic oxide particle-containing resin powder dispersion liquid is a dispersion liquid obtained by dispersing the metallic oxide particle-containing resin powder in a dispersion medium.

Any dispersion media can be used as long as it can disperse the metallic oxide particle-containing resin powder. In addition to water, for example, alcohols, such as methanol, ethanol, 2-propanol, butanol, and octanol; esters, such as ethyl acetate, butyl acetate, ethyl lactate, propylene glycol monomethyl ether acetate, propylene glycol monoethyl ether acetate, and γ-butyrolactone; ethers, such as diethyl ether, ethylene glycol monomethyl ether (methyl-cellosolve), ethylene glycol monethyl ether (ethylcellosolve), ethylene glycol monobutyl ether (butylcellosolve), diethylene glycol monomethyl ether, and diethylene glycol monoethyl ether; ketones, such as acetone, methyl ethyl ketone, methyl isobutyl ketone, acetyl acetone, and cyclohexanone; aromatic hydrocarbons, such as benzene, toluene, xylene, and ethyl-benzene; amides, such as dimethylformamide, N,N-dimethylacetamide, and N-methylpyrrolidone; chain-shaped polysiloxanes, such as dimethylpolysiloxane, methyl-phenyl-polysiloxane, and diphenyl polysiloxane; cyclic polysiloxanes, such as octamethylcyclotetrasiloxane, decamethylcyclopentasiloxane, and dodecamethylcyclohexasiloxane; and modified polysiloxanes, such as amino-modified polysiloxane, polyether-modified polysiloxane, alkyl-modified polysiloxane, and fluorine-modified polysiloxane can be preferably used as the dispersion medium. The solvent can be used singly or as a mixture of two or more kinds.

The dispersion liquid can be obtained by mixing the metallic oxide particle-containing resin powder with the dispersion medium, mixing a dispersant or water-soluble binder with the mixture according to necessity, subsequently, carrying out a dispersion treatment on the mixture using a disperser or mixer, such as a sand mill, a beads mill using zirconia beads, a ball mill, or a homogenizer, and dispersing the metallic oxide particle-containing resin powder in the dispersion medium.

In addition, the necessary time for the dispersion treatment is not particularly limited as long as the time is sufficient for the metallic oxide particle-containing resin powder to be dispersed in the dispersion medium.

When the content of the metallic oxide particle-containing resin powder in the dispersion liquid is adjusted to be 5% by mass, the transmittance T₆₀₀ of the adjusted dispersion liquid with respect to light rays having a wavelength of 600 nm is 60% or more, the ratio T₃₇₅/T₆₀₀ of the transmittance T₃₇₅ with respect to light rays having a wavelength of 375 nm to the transmittance T₆₀₀ with respect to light rays having a wavelength of 600 nm is 0.3 or less, and the ratio T₃₅₀/T₆₀₀ of the transmittance T₃₅₀ with respect to light rays having a wavelength of 350 nm to the transmittance T₆₀₀ with respect to light rays having a wavelength of 600 nm is 0.15 or less.

When the light transmittance of the dispersion liquid is set in the above range, it is possible to secure the transparency with respect to visible light rays.

### [Method of manufacturing metallic oxide particle-containing resin powder]

The method of manufacturing metallic oxide particle-containing resin powder is a method in which metallic oxide particles having an average particle diameter of 0.003 µm to 0.1 µm and a UV shielding function are dispersed in a (meth)acrylic-based resin monomer including 1% by mass to 50% by mass of a dispersant with respect to the metallic oxide particles so as to produce a (meth)acrylic-based resin monomer dispersion liquid, subsequently, the (meth)acrylic-based resin monomer dispersion liquid is suspended or emulsified in pure water including 0.1% by mass to 10% by mass of a suspension protecting agent, 0.01% by mass to 5% by mass of a silicone-based antifoam agent, and 0.1% by mass to 10% by mass of a cross-linking agent with respect to the (meth)acrylic-based resin monomer dispersion liquid so as to produce a suspension liquid or emulsified liquid which contains dispersion particles having a diameter of 0.1 µm to 1 µm, and, subsequently, 0.01% by mass to 1% by mass of a polymerization initiator with respect to the suspension liquid or emulsified liquid is added to the suspension liquid or emulsified liquid so as to cause suspension polymerization or emulsification polymerization, thereby generating metallic oxide particle-containing resin powder.

Here, the method of manufacturing metallic oxide particle-containing resin powder will be described in detail.

Firstly, metallic oxide particles having an average particle diameter of 0.003 µm to 0.1 µm and a UV shielding function are dispersed in a (meth)acrylic-based resin monomer including a dispersant so as to produce a (meth)acrylic-based resin monomer dispersion liquid.

A dispersant that has a high affinity to a resin monomer and is highly hydrophobic is preferred. That is, the dispersant coats the metallic oxide so as to promote dispersion in the resin monomer, a majority of the particles of the metallic oxide turn into a monodispersed state within a relatively short time, and the average dispersion particle diameter becomes 0.003 µm to 0.1 µm.

In addition, since the dispersant makes the metallic oxide particles hydrophobic, the metallic oxide particles do not escape outside, the dispersant does not transit into a water phase, and implantation of the metallic oxide particles in the resin is aided.

Examples of the dispersant include carboxylic acids, such as sodium carboxymethyl cellulose, and salts thereof; sulfonic acids, such as sodium alkanesulfonate, and salts thereof; sulfate esters, such as sodium polyoxyethylene nonylphenyl ether sulfate, and salts thereof; phosphate esters, such as polyoxyethylene alkylphenyl ether phosphate and polyoxyethylene alkyl ether phosphate, and salts thereof; and phosphonic acids, such as sodium lauryl phosphate, and salts thereof.

Particularly, in a case in which the metallic oxide particle-containing resin powder is used for a cosmetic material, the dispersant added should be one of raw materials of the cosmetic material.

The proportion of the dispersant added to the metallic oxide particles is preferably 1% by mass to 50% by mass. When the added proportion is less than 1% by mass, the proportion is too small to cover the surfaces of the metallic oxide particles, and it is not possible to obtain a sufficient dispersion state of the metallic oxide particles. On the other hand, when the proportion exceeds 50% by mass, it is not possible to further improve the dispersibility even in a higher added proportion, and the dispersant is wasted needlessly.

A dispersion apparatus being used is not particularly limited as long as the dispersion apparatus supplies a sufficient dispersion energy to a dispersion system, and examples thereof include a ball mill, a sand mill, an ultrasonic disperser, and a homogenizer.

The dispersion time is preferably approximately 30 minutes to 3 hours, and an appropriate time may be selected in consideration of both the dispersion state and the manufacturing costs.

Thereby, a (meth)acrylic-based resin monomer dispersion liquid in which the average dispersion particle diameter of the metallic oxide particles is 0.003 µm to 0.1 µm can be obtained.

Next, the (meth)acrylic-based resin monomer dispersion liquid is suspended or emulsified in pure water including a suspension protecting agent, a silicone-based antifoaming agent, and a cross-linking agent so as to produce a suspension liquid or emulsified liquid having a dispersion particle diameter of 0.1 µm to 1 µm.

Examples of the suspension protecting agent include non-ionic surfactants, such as polyoxyethylene alkyl ether and polyoxyethylene alkyl phenyl ether; and anionic surfactants, such as alkyl benzene-sulfonate, alkyl sulfate, and alkyl phenyl sulfate. Among them, an anionic surfactant is preferred, and alkyl benzene-sulfonate is preferred as the anionic surfactant.

The amount of the suspension protecting agent added is 0.1% by mass to 10% by mass, and more preferably 0.1% by mass to 2% by mass with respect to the (meth) acrylic-based resin monomer dispersion liquid.

Examples of the silicone-based antifoam agent include an oil type, an oil compound type, a solution type, a powder type, a solid type, an emulsion type, and a self-emulsification type silicone-based antifoam agents and, among them, an oil compound type silicone-based antifoam agent is preferred.

When the silicone-based antifoam agent is added to the (meth)acrylic-based resin monomer dispersion liquid at 0.01% by mass to 5% by mass, the stirring speed of a disperser or mixer can be significantly increased, as a result, the size of the resin powder particles can be decreased to approximately 100 nm, and, when the silicone-based antifoam agent is mixed into a cosmetic material or the like, it is possible to provide a cosmetic material having superior transparency and excellent sensation during use with no roughness. In addition, since the stirring speed of a disperser or mixer can be significantly increased, and, consequently, the efficiency of manufacturing the metallic oxide particle-containing resin powder can be increased, it is possible to significantly reduce the manufacturing costs.

The cross-linking agent is not particularly limited as long as the cross-linking agent is a monomer having two or more unsaturated double bonds, and can be appropriately selected from polyfunctional vinyl monomers, polyfunctional (meth)acrylate derivatives, and the like.

More specifically, examples of the cross-linking agent include (poly) alkylene glycol-based di(meth)acrylates, such as divinyl benzene, divinyl biphenyl, divinyl naphthalene, (poly) ethylene glycol di(meth)acrylate, (poly)propylene glycol di(meth)acrylate, and (poly)tetramethylene glycol di(meth)acrylate.

In addition, examples of the cross-linking agent include alkane diol-based di(meth)acrylates, such as 1,6-hexanediol di(meth)acrylate, 1,8-octanediol di(meth)acrylate, 1,9-nonanediol (meth)acrylate, 1,10-decanediol di(meth)acrylate, 1,12-dodecanediol di(meth)acrylate, 3-methyl-1,5-pentanediol di(meth)acrylate, 2,4-diethyl-1,5-pentanediol di(meth)acrylate, butylethylpropanediol di(meth)acrylate, 3-methyl-1,7-octanediol di(meth)acrylate, and 2-methyl-1,8-octanediol di(meth)acrylate.

In addition, examples of the cross-linking agent include neopentyl glycol di(meth)acrylate, trimethylol propane tri(meth)acrylate, tetramethylol methane tri(meth)acrylate, tetramethylol propane tetra(meth)acrylate, pentaerythritol tri(meth)acrylate, ethoxylated cyclohexane dimethanol di(meth)acrylate, ethoxylated bisphenol A di(meth)acrylate, tricyclodecane dimethanol di(meth)acrylate, propoxylated ethoxylated bisphenol A di(meth)acrylate, 1,1,1-trishydroxy methylethane di(meth)acrylate, 1,1,1-trishydroxy methylethane tri(meth)acrylate, 1,1,1-trishydroxy methylpropane triacrylate, diallyl phthalate and isomers thereof, triallyl isocyanurate and derivatives thereof.

Among them, (poly)ethylene glycol di(meth)acrylate is particularly preferred.

Examples of the polymerization initiator include persulfates, such as potassium persulfate and ammonium persulfate; organic peroxides, such as hydrogen peroxide, benzoyl peroxide, lauroyl peroxide, t-butyl hydroperoxide, and cumene hydroperoxide; and azo-based initiators, such as azobisdiisobutyronitrile, and 2,2-azobis(2-amidinopropane)dihydrochloride. Among them, persulfates are preferred.

When the contents of the suspension protecting agent, the silicone-based antifoam agent, and the polymerization initiator are limited to the above ranges, it is possible to control the average particle diameter of the obtained metallic oxide particle-containing resin powder to 0.1 µm to 1 µm.

In addition, the stirring speed of a disperser or mixer can be significantly increased, consequently, the efficiency of manufacturing the metallic oxide particle-containing resin powder can be increased, and, as a result, it is possible to significantly reduce the manufacturing costs.

Next, a polymerization initiator is added to the suspension liquid or emulsified liquid so as to cause suspension polymerization or emulsification polymerization.

A preferable polymerization method is a method in which the suspension liquid or emulsified liquid is stirred and heated under a nitrogen atmosphere in the presence of a polymerization initiator so as to initiate polymerization.

The polymerization initiating temperature is preferably 50°C to 80°C. In addition, the time during which the suspension liquid or emulsified liquid is polymerized while maintaining the temperature is preferably approximately 1 hour to 5 hours, and an appropriate time may be selected in consideration of a time after which the amount of unreacted residual monomer becomes a minimum, the polymerization state, and the manufacturing costs.

After that, the suspension liquid or emulsified liquid is cooled with ice or naturally cooled, and the polymerization reaction is stopped.

Next, the obtained polymer is sufficiently washed with an alcohol and then washed with pure water in order to remove the remaining monomers, polymerization initiator, and surfactant from the polymer.

Any alcohol may be used as long as the alcohol is soluble in pure water and easily washed off. Examples thereof include ethanol, and 2-propanol, , and 2-propanol is particularly preferred.

The washing method is not particularly limited as long as the remaining monomers and the like can be removed, and the polymer is washed through pressure filtration, suction filtration, filter press, centrifugation, ultrafiltration, decantation, and the like. Washing is carried out until the concentration of 2-propanol becomes 1% or less, and the conductivity becomes 20 µS/cm or less.

After completion of the washing, the obtained polymer is dried at 80°C to 100°C so as to remove the alcohol or pure water, and, subsequently, the obtained polymer is crushed. The drying method is not particularly limited as long as the alcohol or pure water can be removed, and the drying method includes drying in the atmosphere, vacuum drying, and the like.

The crushing method is not particularly limited as long as the obtained polymer can be crushed to 0.1 µm to 1 µm, and examples of the crushing method include a pin mill, a hammer mill, a jet mill, and an impeller mill.

Thereby, the metallic oxide particle-containing resin powder can be generated.

When the metallic oxide particle-containing powder undergoes a crushing process, the respective particles agglomerated by drying are crushed, and the sensation during use can be improved in a case in which the metallic oxide particle-containing powder is used for a cosmetic material.

### [Metallic oxide particle-containing resin powder aqueous dispersion element]

The metallic oxide particle-containing resin powder aqueous dispersion element is an aqueous dispersion element obtained by containing 1% by mass to 80% by mass, more preferably 20% by mass to 70% by mass, and most preferably 30% by mass to 60% by mass of the metallic oxide particle-containing resin powder and containing 5% by mass to 20% by mass of an alcohol.

The aqueous dispersion element may be obtained by further containing 0.001% by mass to 10% by mass, more preferably 0.005% by mass to 5% by mass, and most preferably 0.01% by mass to 3% by mass of the aqueous polymer. In this case, it is necessary to adjust the contents of the respective components so that the contents of the components of the metallic oxide particle-containing resin powder, the alcohol, and the aqueous polymer do not exceed 100% by mass in total.

Examples of the alcohol include monovalent alcohols or multivalent alcohols having a carbon number of 1 to 6, such as ethanol, 1-propanol, 2-propanol, 1-butanol, 2-butanol, glycerin, 1,3-butylene glycol, propylene glycol, and sorbitol, and, among them, a monovalent alcohol, particularly, ethanol is preferred.

In a case in which the aqueous dispersion element does not include the water-soluble polymer, the content of the alcohol is preferably 5% by mass to 20% by mass, and more preferably 10% by mass to 20% by mass.

Particularly, in a case in which the content of the alcohol is set to 10% by mass to 20% by mass, the dispersibility and aging stability of the metallic oxide particle-containing resin powder can be improved, which is preferred.

In addition, in a case in which the aqueous dispersion element includes a water-soluble polymer, the water-soluble polymer is not particularly limited as long as the polymer can be used for cosmetic product use, and examples of the water-soluble polymer include gum arabic, sodium alginate, casein, carrageenan, galactan, carboxy vinyl polymers, carboxy methyl cellulose, sodium carboxymethyl celluose, carboxymethyl starch, agar, xanthan gum, quince seed, guar gum, collagen, gelatin, cellulose, dextran, dextrin, tragacanth gum, hydroxyl ethyl cellulose, hydroxyl propyl cellulose, sodium hyaluronate, pectin, pullulan, methyl cellulose, and methyl hydroxyl propyl cellulose.

The water-soluble polymer has roles of a dispersant and a viscosity adjuster, and improves the dispersibility and aging stability of the metallic oxide particle-containing resin powder when being added.

In a case in which the aqueous dispersion element includes the water-soluble polymer, the content of the alcohol is preferably 5% by mass to 20% by mass, and more preferably 15% by mass to 20% by mass.

Here, the reason why the content of the alcohol is preferably 5% by mass to 20% by mass in a case in which the aqueous dispersion element includes the water-soluble polymer is that, when the content is less than 5% by mass, since the content of the alcohol is too small, the water-soluble polymer cannot homogeneously infiltrate into the alcohol, but inhomogenously swells with moisture, consequently, the dispersibility of the metallic oxide particle-containing resin powder is degraded such that it becomes difficult to handle the metallic oxide particle-containing resin powder, and, furthermore, the aging stability is degraded, which is not preferred. In addition, when the content exceeds 20% by mass, the viscosity of the entire aqueous dispersion element increases, the dispersion stability of the metallic oxide particle-containing resin powder is degraded, and the aging stability is also degraded, which is not preferred.

The metallic oxide particle-containing resin powder aqueous dispersion element can be obtained by mixing the metallic oxide particle-containing resin powder with a solvent containing an alcohol or a mixture including an alcohol and the water-soluble polymer, and, subsequently, mixing and dispersing the obtained mixture with water. The amount of water may be appropriately adjusted, but is preferably in a range of 15% by mass to 94% by mass.

### [Cosmetic material]

The cosmetic material is a cosmetic material containing 1% by mass to 50% by mass, in terms of the metallic oxide particle-containing resin powder, of one or two or more selected from a group of the metallic oxide particle-containing resin powder, the metallic oxide particle-containing resin powder dispersion liquid, and the metallic oxide particle-containing resin powder aqueous dispersion element, and, when the cosmetic material includes the metallic oxide particle-containing resin powder in the above range, there is no concern of whitening, it is possible to sufficiently secure the transparency, furthermore, roughness and the like are not caused, and the sensation during use is excellent.

The cosmetic material can be obtained by mixing one or two or more selected from a group of the metallic oxide particle-containing resin powder, the metallic oxide particle-containing resin powder dispersion liquid, and the metallic oxide particle-containing resin powder aqueous dispersion element in emulsion, cream, foundation, lip sticks, blushes, eye shadow, and the like in the related art manner.

In addition, when one or two or more selected from a group of the metallic oxide particle-containing resin powder, the metallic oxide particle-containing resin powder dispersion liquid, and the metallic oxide particle-containing resin powder aqueous dispersion element are mixed into an aqueous cosmetic material for which formulation was difficult in the related art, such as skin toner and sunscreen gel, elution of the metallic oxide is suppressed, and a cosmetic material that is excellent in terms of a UV shielding function, the transparency, and the sensation during use can be obtained.

In addition, when an organic UV absorbing agent, for example, an azo dye, such as azo-benzene, is added to one or two or more selected from a group of the metallic oxide particle-containing resin powder, the metallic oxide particle-containing resin powder dispersion liquid, and the metallic oxide particle-containing resin powder aqueous dispersion element, it is possible to provide a cosmetic material that is excellent in terms of a UV shielding function and the sensation during use.

Furthermore, when the cosmetic material is used as a component of a cosmetic product, it is possible to provide a variety of cosmetic products, such as skin care cosmetic products, makeup cosmetic products, and body care cosmetic products, which are excellent in terms of the transparency and sensation during use. Particularly, in a case in which the cosmetic material is used for whitening by skin care cosmetic products, base makeup by makeup cosmetic products, screening by body care cosmetic products, and the like, for which a UV shielding function is required, it is possible to provide cosmetic products that are excellent in terms of a UV shielding function, the transparency and sensation during use.

### [Examples]

Hereinafter, the invention will be described specifically using Examples and Comparative Examples, but the invention is not limited to the examples.

### [Example 1]

### [Manufacturing of a resin monomer dispersion liquid]

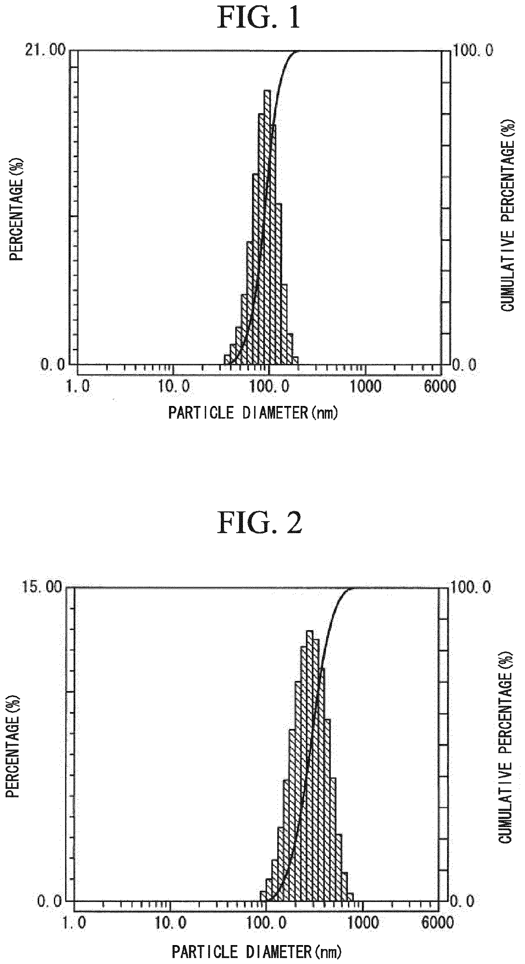

200 parts by mass of zinc oxide fine particles (average particle diameter: 0.02 µm), 188 parts by mass of methyl methacrylate (MMA: resin monomer), and 12 parts by mass of a phosphoric ester surfactant (dispersant) were mixed, and a dispersion treatment was carried out using a sand mill for 2 hours, thereby producing a resin monomer dispersion liquid having the zinc oxide fine particles dispersed in the methyl methacrylate (MMA). As a result of measuring the dispersion particle diameter of the dispersion liquid using a dynamic light scattering particle size distribution measuring apparatus LB-550 (manufactured by Horiba Ltd.), the dispersion particle diameter was 85.0 nm. The volume particle size distribution and cumulative volume particle size distribution are shown in FIG. 1.

105.0 parts by mass of the resin monomer dispersion liquid, 229.5 parts by mass of pure water, 0.5 parts by mass of sodium dodecylbenzenesulfonate, 14.0 parts by mass of ethylene glycol dimethacrylate, and 1.0 part by mass of a silicone-based antifoam agent were mixed and stirred using a homogenizer, thereby producing an emulsion.

By controlling the rotating speed and time of the stirring, the particle size of the emulsion was controlled. Here, the rotating speed and time were controlled under three conditions of (a) 20 minutes of stirring at 10,000 rpm, (b) 20 minutes of stirring at 4,500 rpm, and (c) 20 minutes of stirring at 1,500 rpm so that an emulsion A (10,000 rpm), an emulsion B (4,500 rpm) and an emulsion C (1,500 rpm) were obtained.

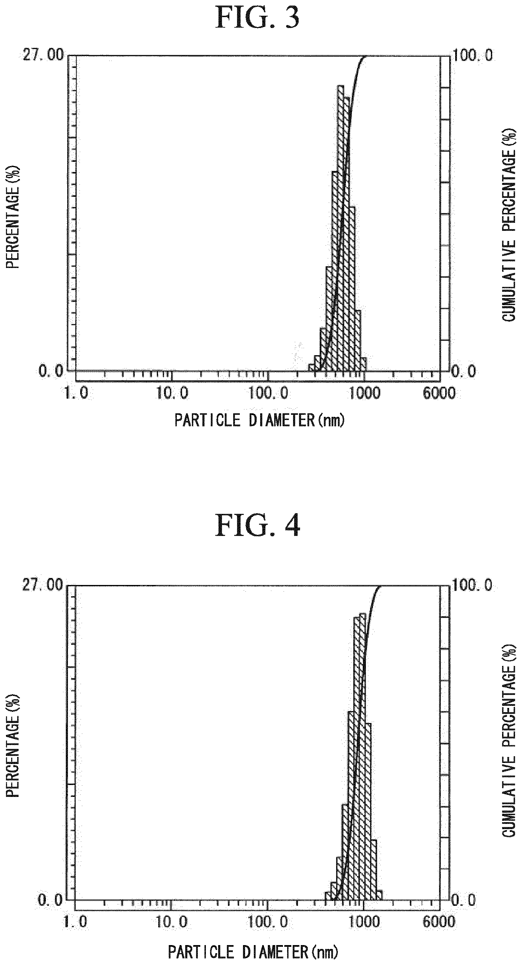

The dispersion particle diameters of the obtained emulsions A to C were measured using a dynamic light scattering particle size distribution measuring apparatus LB-550 (manufactured by Horiba Ltd.). As a result, the dispersion particle diameter of the emulsion A was 300 nm, the dispersion particle diameter of the emulsion B was 600 nm, and the dispersion particle diameter of the emulsion C was 900 nm. The volume particle size distribution and cumulative volume particle size distribution of the emulsion A are shown in FIG. 2, the volume particle size distribution and cumulative volume particle size distribution of the emulsion B are shown in FIG. 3, and the volume particle size distribution and cumulative volume particle size distribution of the emulsion C are shown in FIG. 4, respectively.

### [Manufacturing of resin powder]

1,000.0 parts by mass of each of the emulsions A, B, and C, 249.7 parts by mass of pure water, and 0.3 parts by mass of potassium persulfate were mixed, three obtained mixtures were transferred to a reaction apparatus having a stirrer and a thermometer, and nitrogen substitution was carried out for 1 hour. Subsequently, the mixtures were heated to 65°C, maintained at 65°C for 3 hours, and polymerization reactions were caused. After that, the mixtures were cooled with ice so as to stop the polymerization reactions, the obtained polymers were washed with 2-propanol, furthermore, washed with pure water, and then dried at 90°C. The dried substances obtained in the above manner were crushed using a hammer mill, and three kinds of zinc oxide particle-containing resin powders A, B, and C, which were derived from the emulsions A, B, and C, were obtained.

The dispersion particle diameters of the resin powders A, B, and C were measured using a dynamic light scattering particle size distribution measuring apparatus LB-550 (manufactured by Horiba Ltd.). Here, for each of the zinc oxide particle-containing resin powders A, B, and C, 5 parts by mass of the resin powder was injected into a solution having 10 parts by mass of polyether-modified silicone (dispersant) dissolved in 85% by mass of decamethylcyclopentasiloxane (cyclic silicone), dispersed using a disperser so as to manufacture a dispersion liquid, and the dispersion particle diameter of the resin powder in the dispersion liquid was measured.

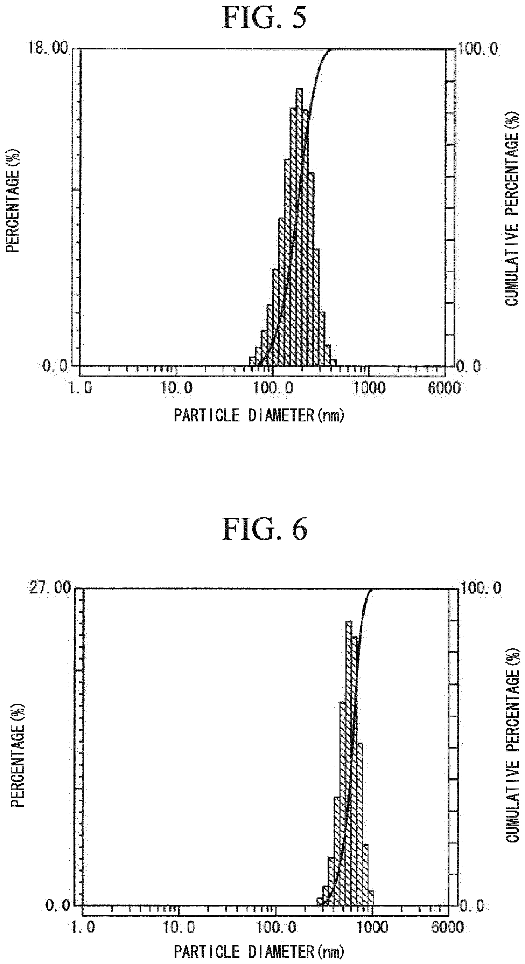

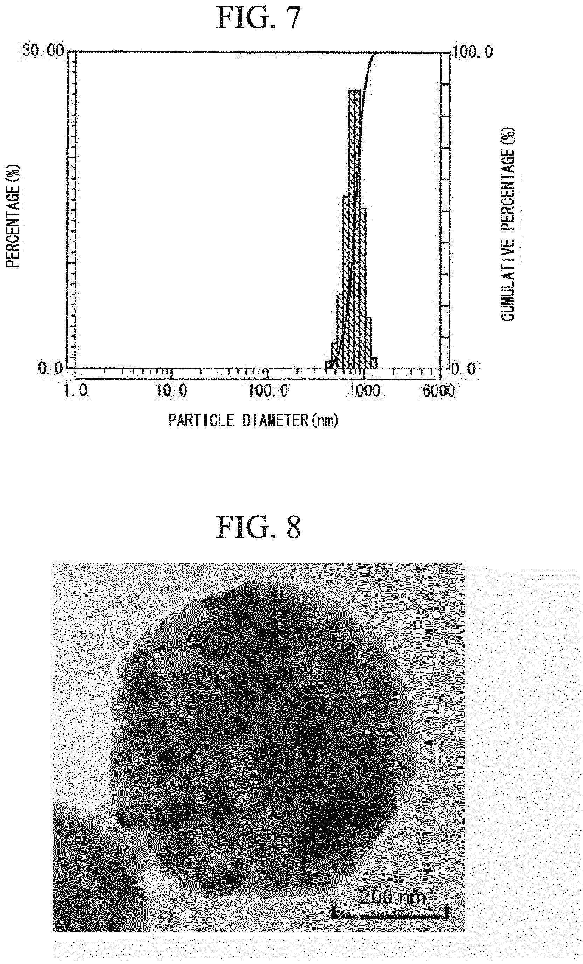

As a result, the dispersion particle diameter of the emulsion A-derived zinc oxide particle-containing resin powder A was 200 nm, the dispersion particle diameter of the emulsion B-derived zinc oxide particle-containing resin powder B was 500 nm, and the dispersion particle diameter of the emulsion C-derived zinc oxide particle-containing resin powder C was 800 nm. The volume particle size distribution and cumulative volume particle size distribution of the zinc oxide particle-containing resin powder A are shown in FIG. 5, the volume particle size distribution and cumulative volume particle size distribution of the zinc oxide particle-containing resin powder B are shown in FIG. 6, and the volume particle size distribution and cumulative volume particle size distribution of the zinc oxide particle-containing resin powder C are shown in FIG. 7, respectively.

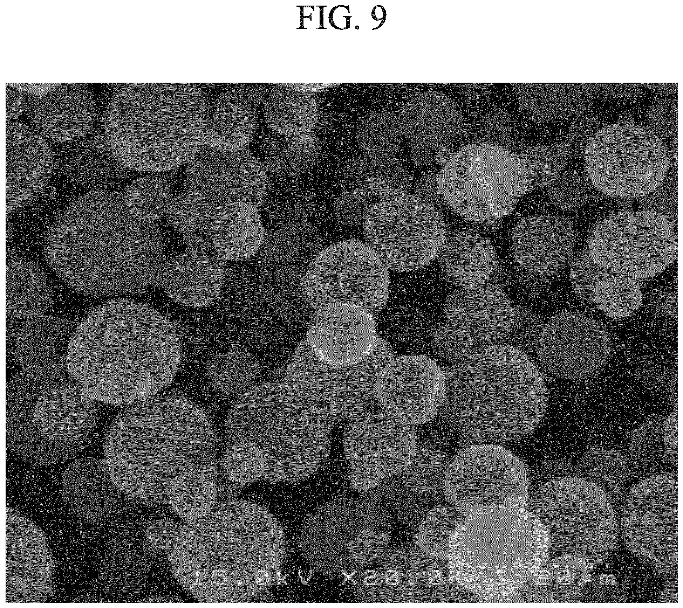

In addition, a transmission electron microscope (TEM) image and a scanning electron microscope (SEM) image of the zinc oxide particle-containing resin powder B are shown in FIGS. 8 and 9 respectively.

### [Comparative Example 1]

105.0 parts by mass of the resin monomer dispersion liquid, which was obtained in Example 1, 229.5 parts by mass of pure water, 0.5 parts by mass of sodium dodecylbenzenesulfonate, and 14.0 parts by mass of ethylene glycol dimethacrylate were mixed and stirred for 20 minutes at 1,500 rpm using a homogenizer, thereby producing an emulsion D.

Meanwhile, of the stirring conditions, under two conditions of 20 minutes of stirring at 10,000 rpm and 20 minutes of stirring at 4,500 rpm, since foam was generated, emulsions could not be obtained.

The dispersion particle diameter of the obtained emulsion D, which was measured using a dynamic light scattering particle size distribution measuring apparatus, was 1,400 nm.

Using the emulsion D, polymerization was carried out based on Example 1, and zinc oxide particle-containing resin powder D, which was derived from the emulsion D, was obtained.

The dispersion particle diameter of the resin powder D, which was measured based on Example 1, was 1,300 nm.

### [Comparative Example 2]

229.5 parts by mass of pure water, 0.5 parts by mass of sodium dodecylbenzenesulfonate, 105.0 parts by mass of methyl methacrylate (MMA: resin monomer), 14.0 parts by mass of ethylene glycol dimethacrylate, and 1.0 part by mass of a silicone-based antifoam agent were mixed, and stirred for 20 minutes using a homogenizer at 4,500 rpm, thereby producing an emulsion E.

The dispersion particle diameter of the obtained emulsion E, which was measured based on Example 1, was 600 nm.

Using the emulsion E, polymerization was carried out based on Example 1, and zinc oxide particle-containing resin powder E, which was derived from the emulsion E, was obtained.

The dispersion particle diameter of the resin powder E, which was measured based on Example 1, was 500 nm.

### [Evaluation of the resin powder]

### (1) Zinc elution test

### Testing method A: elution into pure water (pH 7.4)

0.5 g of each of the zinc oxide particle-containing resin powders A, B, and C, which were obtained in Example 1, was dispersed in 199.5 g of pure water (pH 7.4) using a magnetic stirrer, stirred for 5 minutes, and then left to stand for 1 hour, thereby manufacturing three types of test specimens. After that, the whitened supernatants of the three types of test specimens were taken, and sedimentation separation was carried out for 1 hour at 21,000 rpm using a centrifugal machine, thereby collecting transparent supernatants. The collected supernatants were filtered using a filter (mesh: 0.025 µm), and the weight of eluted zinc in the filtered liquid was determined by the atomic absorption method.

Here, as Comparative Examples, the zinc oxide particle-containing resin powder D, which was obtained in Comparative Example 1, untreated zinc oxide, and surface-treated zinc oxide, on which a silica/silicon treatment was carried out, were treated in the same manner, and the weight of eluted zinc was determined.

Table 1 shows the measurement results.

**[Table 1]**

| Kind of particles | Amount of zinc eluted (ppm) |
|---|---|
| Resin powder A | 0.001 |
| Resin powder B | 0.001 |
| Resin powder C | 0.003 |
| Resin powder D | 0.032 |
| Untreated zinc oxide | 1.878 |
| Silica/silicone-treated zinc oxide | 0.079 |

### Testing method B: elution into diluted acetic acid (pH 5.0)

0.5 g of each of the zinc oxide particle-containing resin powders A, B, and C, which were obtained in Example 1, was dispersed in 199.5 g of diluted acetic acid (pH 5.0) using a magnetic stirrer, stirred for 5 minutes, and then left to stand for 1 hour, thereby manufacturing three types of test specimens. After that, the whitened supernatants of the three types of test specimens were taken, and sedimentation separation was carried out for 1 hour at 21, 000 rpm using a centrifugal machine, thereby collecting transparent supernatants. The collected supernatants were filtered using a filter (mesh: 0.025 µm), and the weight of eluted zinc in the filtered liquid was determined by the atomic absorption method.

Here, as Comparative Examples, the zinc oxide particle-containing resin powder D, which was obtained in Comparative Example 1, untreated zinc oxide, and surface-treated zinc oxide, on which a silica/silicon treatment was carried out, were treated in the same manner, and the weight of eluted zinc was determined.

Table 2 shows the measurement results.

**[Table 2]**

| Kind of particles | Amount of zinc eluted (ppm) |
|---|---|
| Resin powder A | 0.427 |
| Resin powder B | 0.586 |
| Resin powder C | 0.632 |
| Resin powder D | 1.721 |
| Untreated zinc oxide | 4.747 |
| Silica/silicone-treated zinc oxide | 2.810 |

### (2) Spectral transmittance

The zinc oxide particle-containing resin powders A, B, and C, which were obtained in Example 1, and the resin powders D and E, which were obtained in Comparative Examples 1 and 2 were mixed in a ratio of 5.0 parts by mass of the resin powder, 90.0 parts by mass of decamethylcyclopentasiloxane (cyclic silicone), and 5.0 parts by mass of polyether-modified silicone, a dispersion treatment was carried out using a sand mill for 2 hours at 2,500 rpm, thereby producing dispersion liquids A to E of the resin powders A to E.

Subsequently, each of the dispersion liquids A to E was accommodated in a 50 µm spectrophotometer cell, and the spectral transmittance was measured using a spectrophotometer.

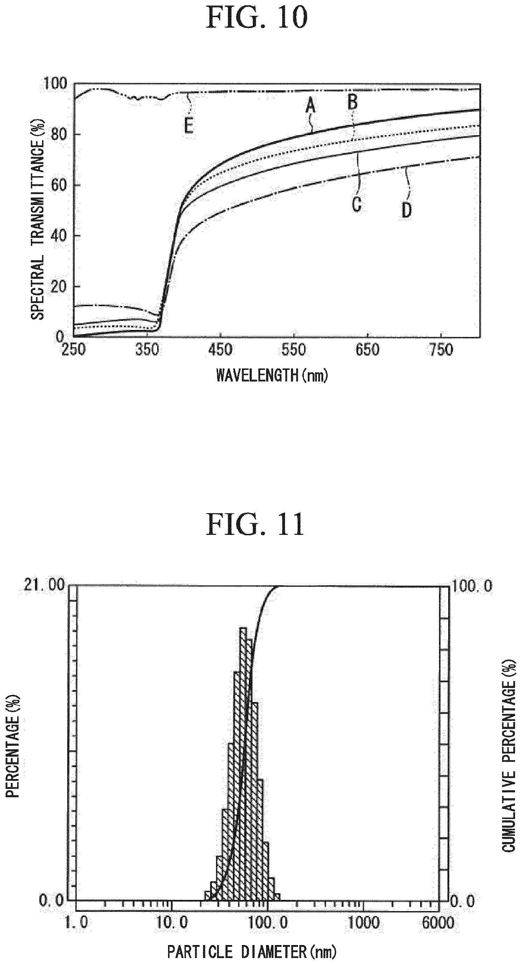

FIG. 10 shows the spectral transmittance of the respective dispersion liquids A to E.

### [Example 2]

### [Manufacturing of a resin monomer dispersion liquid]

120 parts by mass of titanium oxide fine particles (average particle diameter: 0.02 µm), 256 parts by mass of methyl methacrylate (MMA: resin monomer), and 24 parts by mass of a phosphoric ester surfactant (dispersant) were mixed, and a dispersion treatment was carried out using a sand mill for 2 hours, thereby producing a resin monomer dispersion liquid having the titanium oxide fine particles dispersed in the methyl methacrylate (MMA). As a result of measuring the dispersion particle diameter of the dispersion liquid using a dynamic light scattering particle size distribution measuring apparatus, the dispersion particle diameter was 55 nm. The volume particle size distribution and cumulative volume particle size distribution are shown in FIG. 11.

### [Manufacturing of emulsion]

105.0 parts by mass of the resin monomer dispersion liquid, 229.5 parts by mass of pure water, 0.5 parts by mass of sodium dodecylbenzenesulfonate, 14.0 parts by mass of ethylene glycol dimethacrylate, and 1.0 part by mass of a silicone-based antifoam agent were mixed and stirred for 20 minutes using a homogenizer at 4,500 rpm, thereby producing an emulsion E.

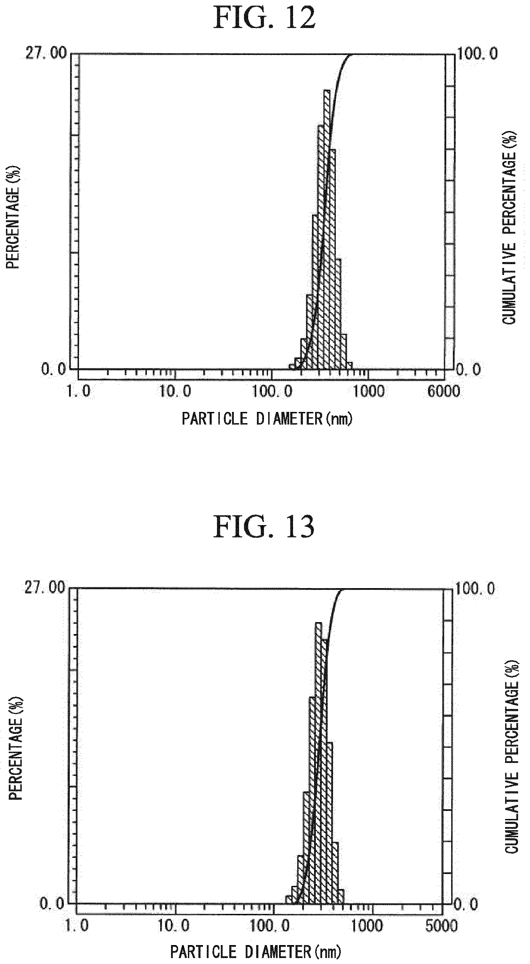

The dispersion particle diameter of the emulsion E, which was measured using a dynamic light scattering particle size distribution measuring apparatus, was 350 nm. The volume particle size distribution and cumulative volume particle size distribution of the emulsion E are shown in FIG. 12.

### [Manufacturing of resin powder]

1,000.0 parts by mass of the emulsion E, 249.7 parts by mass of pure water, and 0.3 parts by mass of potassium persulfate were mixed, the obtained mixture was transferred to a reaction apparatus having a stirrer and a thermometer, and nitrogen substitution was carried out for 1 hour. Subsequently, the mixtures were heated to 65°C, maintained at 65°C for 3 hours, and polymerization reactions were caused. After that, the mixtures were cooled with ice so as to stop the polymerization reactions, the obtained polymers were washed with 2-propanol, furthermore, washed with pure water, and then dried at 90°C. The dried substance obtained in the above manner was crushed using a hammer mill, and titanium oxide particle-containing resin powder E was obtained.

The dispersion particle diameter of the titanium oxide particle-containing resin powder E, which was measured based on Example 1, was 280 nm. The volume particle size distribution and cumulative volume particle size distribution of the titanium oxide particle-containing resin powder E are shown in FIG. 13.

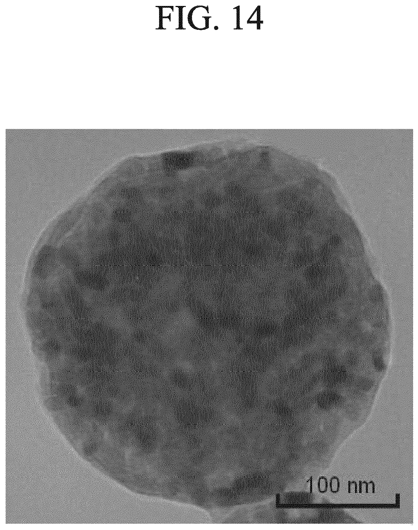

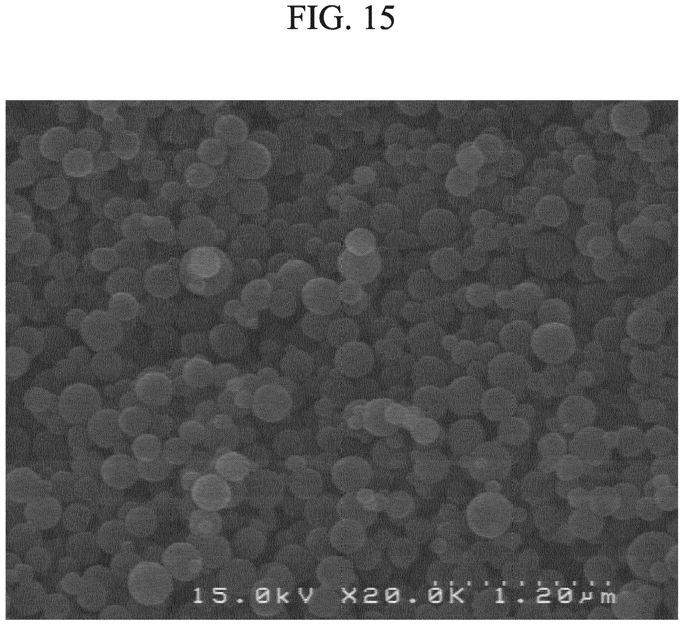

In addition, a transmission electron microscope (TEM) image and a scanning electron microscope (SEM) image of the titanium oxide particle-containing resin powder E are shown in FIGS. 14 and 15 respectively.

### [Example 3]

### [Manufacturing of a resin monomer dispersion liquid]

160 parts by mass of cerium oxide fine particles (average particle diameter: 0.03 µm), 228 parts by mass of methyl methacrylate (MMA: resin monomer), and 12 parts by mass of a phosphoric ester surfactant (dispersant) were mixed, and a dispersion treatment was carried out using a sand mill for 2 hours, thereby producing a resin monomer dispersion liquid having the titanium oxide fine particles dispersed in the methyl methacrylate (MMA). As a result of measuring the dispersion particle diameter of the dispersion liquid based on Example 1, the dispersion particle diameter was 80.0 nm.

### [Manufacturing of resin powder]

Using the resin monomer dispersion liquid, an emulsion was obtained based on Example 1.

Here, the mixture was stirred for 20 minutes at 4,500 rpm.

Next, using the emulsion, cerium oxide particle-containing resin powder was obtained based on Example 1.

The dispersion particle diameter of the resin powder, which was measured based on Example 1, was 400 nm.

### [Example 4]

### [Manufacturing of a resin monomer dispersion liquid]

160 parts by mass of iron oxide fine particles (average particle diameter: 0.05 µm), 228 parts by mass of methyl methacrylate (MMA: resin monomer), and 12 parts by mass of a phosphoric ester surfactant (dispersant) were mixed, and a dispersion treatment was carried out using a sand mill for 2 hours, thereby producing a resin monomer dispersion liquid having the titanium oxide fine particles dispersed in the methyl methacrylate (MMA). As a result of measuring the dispersion particle diameter of the dispersion liquid based on Example 1, the dispersion particle diameter was 95.0 nm.

### [Manufacturing of resin powder]

Using the resin monomer dispersion liquid, an emulsion was obtained based on Example 1.

Here, the mixture was stirred for 20 minutes at 4,500 rpm.

Next, using the emulsion, iron oxide particle-containing resin powder was obtained based on Example 1.

The dispersion particle diameter of the resin powder, which was measured based on Example 1, was 500 nm.

### [Example 5]

### [Manufacturing of a resin monomer dispersion liquid]

120 parts by mass of zinc oxide fine particles (average particle diameter: 0.02 µm), 80 parts by mass of titanium oxide fine particles (average particle diameter: 0.04 µm), 188 parts by mass of methyl methacrylate (MMA: resin monomer), and 12 parts by mass of a phosphoric ester surf actant (dispersant) were mixed, and a dispersion treatment was carried out using a sand mill for 2 hours, thereby producing a resin monomer dispersion liquid having the zinc oxide fine particles and the titanium oxide fine particles dispersed in the methyl methacrylate (MMA). As a result of measuring the dispersion particle diameter of the dispersion liquid using a dynamic light scattering particle size distribution measuring apparatus, the dispersion particle diameter was 80.0 nm.

### [Manufacturing of resin powder]

Using the resin monomer dispersion liquid, an emulsion was obtained based on Example 1.

Here, the mixture was stirred for 20 minutes at 4,500 rpm.

Next, using the emulsion, zinc oxide particle and titanium oxide particle-containing resin powder was obtained based on Example 1.

The dispersion particle diameter of the resin powder, which was measured based on Example 1, was 500 nm.

### [Example 6]

### [Manufacturing of a rein powder aqueous dispersion element]

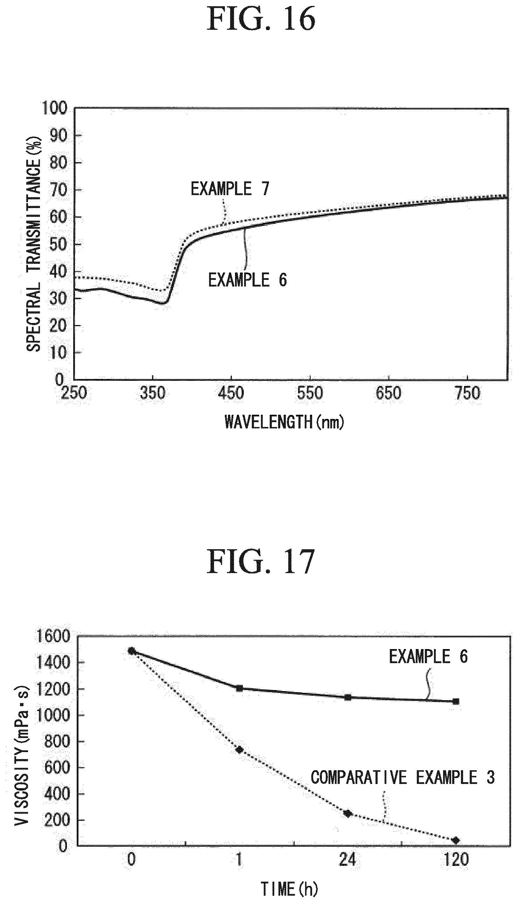

150 parts by mass of ethanol, 450 parts by mass of pure water, and 400 parts by mass of the zinc oxide particle-containing resin powder B of Example 1 were sufficiently stirred and mixed using a homogenizer so as to produce zinc oxide particle-containing resin powder aqueous dispersion element, and the spectral transmittance of the aqueous dispersion element was measured based on Example 1. The spectral transmittance of the resin powder aqueous dispersion element is shown in FIG. 16.

Next, the viscosity of the resin powder aqueous dispersion element was measured.

Here, 25 parts by mass of the obtained aqueous dispersion element, 25 parts by mass of pure water, and 50 parts by mass of crystal gel (aqueous gel: manufactured by Pinoa) were sufficiently mixed using a homogenizer, and the viscosity of the mixture immediately after the mixing was measured using a B-type viscometer (manufactured by Toki Sangyo Co., Ltd.). After that, the mixture was left to stand at 25°C for 120 hours, and the viscosity of the mixture immediately after the 120 hours of being left to stand was measured using a B-type viscometer (manufactured by Toki Sangyo Co., Ltd.). The change of the viscosity between immediately after the mixing and after the 120 hours of being left to stand was 25.6%. The measurement results of the viscosity are shown in FIG. 17.

### [Example 7]

20 parts by mass of carboxy-methyl cellulose and 50 parts by mass of ethanol were added to 530 parts by mass of pure water, the mixture was stirred and sufficiently dissolved using a Food Mixer (Kenwood KMM770 Chef Major Premier Mixer), then, 400 parts by mass of the zinc oxide particle-containing resin powder B of Example 1 was added, again, sufficiently stirred and mixed using the Food Mixer, thereby producing a zinc oxide fine particle-containing resin powder aqueous dispersion element. Next, the spectral transmittance of the aqueous dispersion element was measured based on Example 1. The spectral transmittance of the resin powder aqueous dispersion element is shown in FIG. 16.

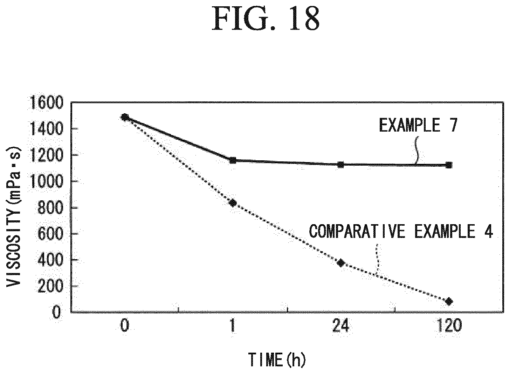

Next, the viscosity of the zinc oxide fine particle aqueous dispersion element was measured based on Example 6. As a result, The change of the viscosity of the zinc oxide fine particle aqueous dispersion element between immediately after the mixing and after the 120 hours of being left to stand was 24.2%. The measurement results of the viscosity are shown in FIG. 18.

### [Comparative Example 3]

The zinc oxide fine particle aqueous dispersion element of Comparative Example 3 was obtained based on Example 6 except that zinc oxide fine particles (average particle diameter 0.02 µm) was used instead of the zinc oxide particle-containing resin powder B in Example 6.

Next, the viscosity of the zinc oxide fine particle aqueous dispersion element was measured based on Example 6. As a result, The change of the viscosity of the zinc oxide fine particle aqueous dispersion element between immediately after the mixing and after the 120 hours of being left to stand was extremely large, 96.5%. The measurement results of the viscosity are shown in FIG. 17.

### [Comparative Example 4]

The zinc oxide fine particle aqueous dispersion element of Comparative Example 4 was obtained based on Example 7 except that zinc oxide fine particles (average particle diameter 0.02 µm) was used instead of the zinc oxide particle-containing resin powder B in Example 7.

Next, the viscosity of the zinc oxide fine particle aqueous dispersion element was measured based on Example 6. As a result, The change of the viscosity of the zinc oxide fine particle aqueous dispersion element between immediately after the mixing and after the 120 hours of being left to stand was extremely large, 94.4%. The measurement results of the viscosity are shown in FIG. 18.

### Industrial Applicability

Since the metallic oxide particle-containing resin powder obtained with the method of the invention can be made into fine particles, improve the UV absorbing performance, the transparency with respect to visible light rays. In addition, since the metallic oxide particles are not exposed on the surface of the resin powder, even in a case in which the resin powder is dispersed in the solvent, it is possible to suppress elution of a metallic oxide, which is a component of the particles, into a solvent. Therefore, the resin powder can be applied not only to water-in-oil type (W/O type) cosmetic products but also to oil-in-water type (O/W type) cosmetic products.

## Claims

1. A method of manufacturing metallic oxide particle-containing resin powder, including:
dispersing metallic oxide particles having an average particle diameter of 0.003 µm to 0.1 µm and a UV shielding function in a (meth)acrylic-based resin monomer including 1% by mass to 50% by mass of a dispersant with respect to the metallic oxide particles to produce a (meth)acrylic-based resin monomer dispersion liquid;
suspending or emulsifying the (meth)acrylic-based resin monomer dispersion liquid in pure water including 0.1% by mass to 10% by mass of a suspension protecting agent, 0.01% by mass to 5% by mass of a silicone-based antifoam agent, and 0.1% by mass to 10% by mass of a cross-linking agent with respect to the (meth)acrylic-based resin monomer dispersion liquid to produce a suspension liquid or emulsified liquid; and,
adding 0.01% by mass to 1% by mass of a polymerization initiator with respect to the suspension liquid or emulsified liquid to the suspension liquid or emulsified liquid to cause suspension polymerization or emulsification polymerization, thereby generating metallic oxide particle-containing resin powder.

2. Method of manufacturing metallic oxide particle-containing resin powder according to Claim 1, wherein the metallic oxide particles having a UV shielding function is particles that include zinc oxide particles.

## Patentansprüche

1. Ein Verfahren zur Herstellung eines Harzpulvers, das darin eingekapselte Metalloxidpartikel enthält, einschließlich:
Dispersion von Metalloxidpartikeln, mit einem mittleren Partikeldurchmesser von 0,003 µm bis 0,1 µm und einer Ultraviolett-Abschirmfunktion in einem Harz-Monomer auf (Meth)-acrylbasis, einschließlich 1 bis 50 Masse-% eines Dispergiermittels, bezogen auf die Metalloxidpartikel, zur Erzeugung einer Harz-Monomerflüssigkeit auf (Meth)-acrylbasis;
Suspendieren oder Emulgieren der Harz-Monomerflüssigkeit auf (Meth)-acrylbasis in reinem Wasser, einschließlich 0,1 Masse-% bis 10 Masse-% eines Suspensionsschutzmittels, 0,01 Masse-% bis 5 Masse-% eines Antischaummittels auf Silikonbasis und 0,1 Masse-% bis 10 Masse-% eines Vernetzungsmittels, bezogen auf die Harz-Monomerflüssigkeit auf (Meth)-acrylbasis, zur Erzeugung einer Suspensions- oder Emulsionsflüssigkeit; und
Zugabe von 0,01 Masse-% bis 1 Masse-% eines Polymerisationsinitiators bezogen auf die Suspensions- oder Emulsionsflüssigkeit zu der Suspensions- oder Emulsionsflüssigkeit, um eine Suspensionspolymerisation oder Emulsionspolymerisation zu verursachen und dabei Metalloxidpartikel enthaltendes Harzpulver zu erzeugen.

2. Verfahren zur Herstellung eines Harzpulvers, das darin eingekapselte Metalloxidpartikel enthält, gemäß Anspruch 1, wobei die Metalloxidteilchen, die eine UV-Abschirmfunktion haben, Teilchen sind, die Zinkoxidteilchen enthalten.

## Revendications

1. Procédé de fabrication d'une poudre de résine contenant des particules d'oxyde métallique, incluant :
la dispersion de particules d'oxyde métallique ayant un diamètre moyen de particule de 0,003 µm à 0,1 µm et un effet d'écran vis-à-vis des ultraviolets dans un monomère de résine à base (méth)acrylique incluant de 1 % en masse à 50 % en masse d'un dispersant par rapport aux particules d'oxyde métallique pour produire une dispersion liquide de monomère de résine à base (méth)acrylique ;
la mise en suspension ou l'émulsification de la dispersion liquide de monomère de résine à base (méth)acrylique dans de l'eau pure incluant de 0,1 % en masse à 10 % en masse d'un agent protecteur en suspension, de 0,01 % en masse à 5 % en masse d'un agent antimousse à base de silicone et de 0,1 % en masse à 10 % en masse d'un agent de réticulation par rapport à la dispersion liquide de monomère de résine à base (méth)acrylique pour produire une suspension liquide ou un liquide émulsifié et
l'ajout de 0,01 % en masse à 1 % en masse d'un initiateur de polymérisation par rapport à la suspension liquide ou au liquide émulsifié à la suspension liquide ou au liquide émulsifié pour provoquer une polymérisation en suspension ou une polymérisation-émulsification, en générant ainsi une poudre de résine contenant des particules d'oxyde métallique.

2. Procédé de fabrication d'une poudre de résine contenant des particules d'oxyde métallique selon la revendication 1, dans lequel les particules d'oxyde métallique ayant un effet d'écran vis-à-vis des ultraviolets sont des particules qui incluent des particules d'oxyde de zinc.
